# EUROPÄISCHE PATENTSCHRIFT

(11) **EP 0 902 013 B1**
(45) Veröffentlichungstag und Bekanntmachung des Hinweises auf die Patenterteilung: **12.12.2001**
(21) Anmeldenummer: 98121305.1
(22) Anmeldetag: 14.01.1993
(51) Int. Cl.: C07C 257/18, A61K 31/155

(54) **Neue Amiderivate, ihre Herstellung und Verwendung als Arzneimittel mit LTB4-antagonistischer Wirkung**
Novel amidine derivatives, their preparation and their use as medicaments with LTB-4 antagonistic effect
Nouveau dérivés de l'amidine, leur fabrication et leur application en tant que médicaments ayant un effet antagoniste à LTB4

(30) Priorität: 05.02.1992 DE 4203201; 23.07.1992 DE 4224289; 24.12.1992 DE 4244241
(43) Veröffentlichungstag der Anmeldung: 17.03.1999
(62) Teilanmeldung aus: 93902195.2
(73) Patentinhaber: Boehringer Ingelheim International GmbH, 55218 Ingelheim am Rhein (DE); Boehringer Ingelheim Pharma KG, 55218 Ingelheim am Rhein (DE)
(72) Erfinder: Anderskewitz, Ralf, 55411 Bingen am Rhein (DE); Schromm, Kurt, 55218 Ingelheim am Rhein (DE); Renth, Ernst-Otto, 24105 Kiel (DE); Himmelsbach, Frank, 88441 Mittelbiberach (DE); Birke, Franz, 55218 Ingelheim am Rhein (DE); Fügner, Armin, 55435 Gau-Algesheim (DE)

(56) Entgegenhaltungen:
- EP-A- 0 292 977
- EP-A- 0 366 066
- EP-A- 0 518 818
- EP-A- 0 518 819
- C. HANSCH ET AL: "STRUCTURE-ACTIVITY RELATIONSHIP IN IMMUNOCHEMISTRY.2. INHIBITION OF COMPLEMENT BY BENZAMIDES" JOURNAL OF MEDICINAL CHEMISTRY, Bd. 17, Nr. 11, 1974, Seite 1160 XP002087330 WASHINGTON US
- B.R. BAKER ET AL.: "IRREVERSIBLE ENZYME INHIBITORS. CLII. PROTEOLYTIC ENZYMES. X. INHIBITION OF GUINEA PIG COMPLEMENT BY SUBSTITUTED BENZAMIDINES" JOURNAL OF MEDICINAL CHEMISTRY, Bd. 12, 1969, Seiten 408-414, XP002087331 WASHINGTON US

## Beschreibung

Die Erfindung betrifft neue Amidinderivate, ihre Herstellung nach konventionellen Methoden und ihre Verwendung in Arzneimitteln.

Die erfindungsgemäßen Verbindungen sind therapeutisch verwendbar, insbesondere aufgrund ihrer LTB₄-antagonistischen Wirkung. Die breite allgemeine Formel der europäischen Patentanmeldung EP 0 518 818 umfasst zwar die erfindungsgemäßen Benzamidine zum Teil. Jedoch findet sich dort keinerlei Hinweis auf solche Benzamidine, die eine 2-n-Propyl-3-alkoxyphenylgruppe aufweisen.

Die neuen Amidinderivate entsprechen der Formel 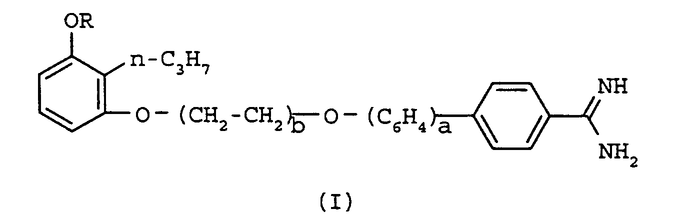 worin
a 0 oder 1,
b 1 oder 2, und
R Wasserstoff oder C₁-C₄-Alkyl bedeuten, mit der Maßgabe, dass im Falle a = 0 und b = 2, R nur C₁-C₄-Alkyl bedeutet,
und können (wenn sie ein chirales Zentrum enthalten) als Racemate, in enantiomerenreiner bzw. angereicherter Form, jeweils als freie Basen oder als Salze, vorzugsweise mit physiologisch verträglichen Säuren, vorliegen.

Bevorzugt sind (im Rahmen der obigen Definitionen) Verbindungen der Formel I, worin
a = 0 oder 1/ b = 1 oder a = 0/b = 2 ist, insbesondere wobei R H, CH₃ oder C₂H₅, a 0 oder 1 und b 1 bedeutet, oder wobei a 0, b 2, R CH₃ bedeutet.

Als Gruppe der Formel 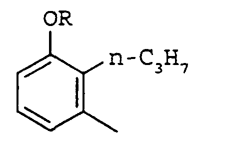 seien besonders hervorgehoben:

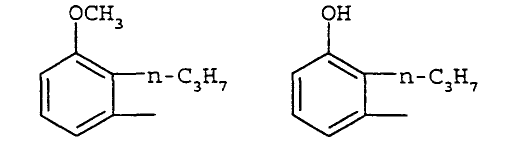

Soweit die aufgeführten Gruppen Alkylketten sind oder solche enthalten, können diese geradkettig oder verzweigt sein.

Die neuen Verbindungen werden nach konventionellen Methoden hergestellt.

### 1. Umsetzung von Imidoestern der Formel

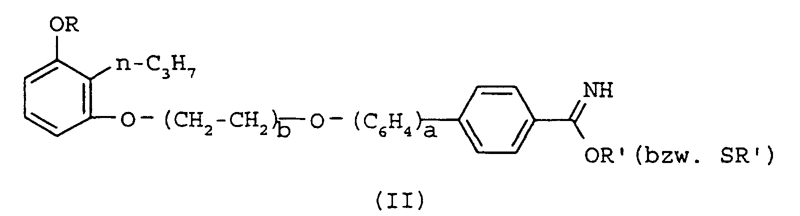

worin R' C₁-C₆-Alkyl oder Benzyl bedeutet,
und a, b und R die oben angegebene Bedeutung aufweisen, mit Ammoniak.

Die Umsetzung erfolgt zweckmäßig in einem organischen Lösungsmittel bei Temperaturen zwischen etwa 0°C und der Siedetemperatur des Reaktionsgemischs, vorzugsweise zwischen Raumtemperatur und etwa 100°C bzw. der Siedetemperatur, soweit diese niedriger ist. Geeignete Lösungsmittel sind polare Lösungsmittel wie Methanol, Ethanol, Propanole.
Bei hinreichend säurestabilen Ausgangsstoffen kann die Umsetzung statt über die Imidoester auch über die entsprechenden Säureimidchloride erfolgen.

### 2. Umsetzung von Phenolen bzw. Biphenylolen

### 1) Umsetzung von Phenolen der Formel

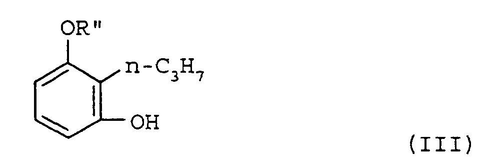

worin
- R": C₁-C₄-Alkyl bedeutet, mit einer Verbindung der Formel 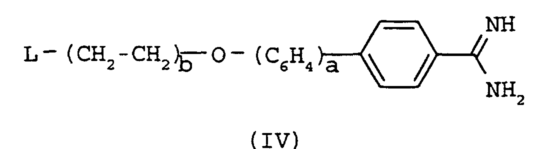 worin a und b die obige Bedeutung haben und L für eine nucleofuge Abgangsgruppe steht bzw.

### 2) Umsetzung von Phenolen der Formel

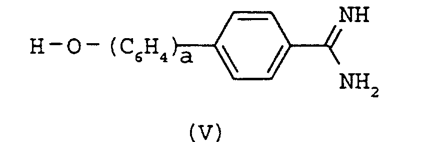

worin a die obige Bedeutung hat, mit einer

Verbindung der Formel 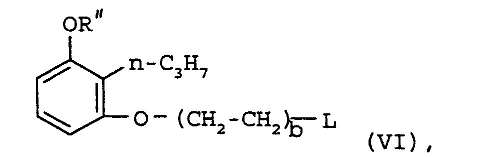 worin b, L und R" die obige Bedeutung haben.

Die Umsetzung erfolgt in aprotischen Lösungsmitteln wie Dimethylsulfoxid, Dimethylformamid, Acetonitril oder Alkoholen wie Methanol, Ethanol oder Propanol unter Zusatz einer Base (Metallcarbonate, Metallhydroxide, Metallhydride) bei Temperaturen zwischen etwa 0 und 140°C bzw. der Siedetemperatur des Reaktionsgemischs.

Die Phenole können auch in Form von Salzen, etwa der Alkalisalze, eingesetzt werden. Als nucleofuge Abgangsgruppe eignen sich z.B. Halogene, etwa Br, Cl.

### 3. Reduktion eines Amidoxims der Formel

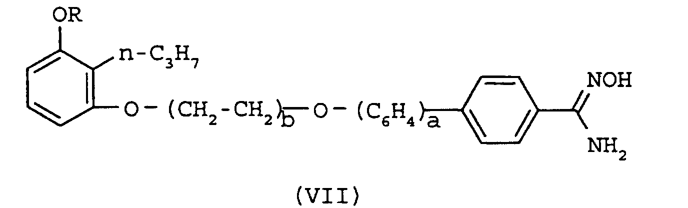

worin R, a und b die obige Bedeutung haben.

Für die Stufe der Reduktion von VII eignet sich die katalytische Hydrierung, insbesondere mit Raney-Nickel in einem niederen Alkohol, z.B. Methanol. Zweckmäßig wird das Amidoxim der Formel VII unter Zugabe der berechneten Menge derjenigen Säure, deren Salz als Endprodukt gewünscht wird, in Methanol gelöst und bei Raumtemperatur unter leichtem Druck, z.B. bei 5 bar, bis zur beendeten Wasserstoffaufnahme hydriert.

Die Ausgangsstoffe können nach üblichen Methoden aus bekannten Verbindungen erhalten werden.

So können die Ausgangsstoffe für Verfahren 1 aus den entsprechenden Nitrilen durch Umsetzung mit HCl über die Stufe der Imidchloride bzw. direkt durch Umsetzung mit z.B. C₁-C₆-Alkoholen bzw. Benzylalkohol in Gegenwart einer Säure wie HCl erhalten werden. Auch die Umsetzung der Nitrile mit H₂S in Lösungsmitteln wie Pyridin oder Dimethylformamid in Gegenwart einer Base wie Triethylamin und anschließende Alkylierung bzw. Benzylierung führen zu Verbindungen der Formel II. Ausgehend von Carbonsäureamiden, die im übrigen den Verbindungen der Formel II entsprechen, gelangt man auch durch Umsetzung mit einem Trialkyloxoniumsalz wie Triethyloxoniumtetrafluoroborat in einem Lösungsmittel wie Dichlormethan, Tetrahydrofuran oder Dioxan bei Temperaturen zwischen 0 und 50°C, vorzugsweise bei Raumtemperatur zu Verbindungen der Formel II.

Für die Herstellung der Ausgangsstoffe VII können auch Umsetzungen entsprechender Amidoxime anstelle von Amidinen analog Verfahren 1 oder 2 dienen; durch analoge oder Umsetzung entsprechender Nitrile, aus denen abschließend durch Addition von Hydroxylamin die Ausgangsstoffe VII entstehen.

Die erfindungsgemäßen Verbindungen sind therapeutisch verwendbar, insbesondere aufgrund ihrer LTB₄-antagonistischen Wirkung. Sie eignen sich daher für die Anwendung vor allem bei solchen Krankheiten, bei denen entzündliche und/oder allergische Vorgänge eine Rolle spielen, beispielsweise, Asthma, Colitis ulcerosa, Psoriasis, ferner zur Behandlung einer durch nichtsteroidale Antiphlogistika induzierten Gastropathie. Die neuen Verbindungen können auch in Kombination mit anderen Wirkstoffen angewendet werden, z. B. mit Antiallergika, Sekretolytika, β₂-Adrenergika, inhalativ anwendbaren Steroiden, Antihistaminika und/oder PAF-Antagonisten. Die Verabreichung kann topisch, oral, transdermal, nasal, parenteral oder inhalativ erfolgen.

Die therapeutische oder prophylaktische Dosis ist - außer von der Wirkungsstärke der einzelnen Verbindungen und dem Körpergewicht des Patienten - abhängig von der Beschaffenheit und Ernsthaftigkeit des Krankheitszustandes. Bei oraler Anwendung liegt die Dosis zwischen 10 und 250 mg, vorzugsweise zwischen 20 und 200 mg. Bei inhalativer Anwendung werden dem Patienten zwischen etwa 2 und 20 mg Wirkstoff zugeführt.
Die neuen Verbindungen können in üblichen Zubereitungen verabreicht werden, etwa als Tabletten, Dragées, Kapseln, Oblaten, Pulver, Granulate, Lösungen, Emulsionen, Sirupe, Inhalationsaerosole, Salben, Suppositorien.

Die nachstehenden Beispiele zeigen einige Möglichkeiten für die Formulierung der Darreichungsformen.

### Formulierungsbeispiele

1. Tabletten

| Zusammensetzung: | |
|---|---|
| Wirkstoff gemäß der Erfindung | 20 Gew.-Teile |
| Stearinsäure | 6 Gew.-Teile |
| Traubenzucker | 474 Gew.-Teile |

Die Bestandteile werden in üblicher Weise zu Tabletten von 500 mg Gewicht verarbeitet. Gewünschtenfalls kann der Wirkstoffgehalt erhöht oder vermindert und die Traubenzuckermenge entsprechend vermindert oder erhöht werden.
2. Suppositorien

| Zusammensetzung: | |
|---|---|
| Wirkstoff gemäß der Erfindung | 100 Gew.-Teile |
| Laktose, gepulvert | 45 Gew.-Teile |
| Kakao-Butter | 1555 Gew.-Teile |

Die Bestandteile werden in üblicher Weise zu Suppositorien von 1,7 g Gewicht verarbeitet.
3. Inhalationspulver
Mikronisiertes Wirkstoffpulver (Verbindung der Formel I; Teilchengröße ca. 0,5 bis 7 µm) werden in einer Menge von 5 mg gegebenenfalls unter Zusatz mikronisierter Lactose in Hartgelatinekapseln abgefüllt. Das Pulver wird aus üblichen Inhalationsgeräten, z.B. gemäß DE-A 3 345 722, inhaliert.
Die erfindungsgemäßen Verbindungen wurden u.a. auf ihre Wirkung in den nachstehenden Testen untersucht.

### a) U937 - Rezeptorverbindungstest/LTB₄

Die Bindung von ³H-LTB₄ (3nM) auf vitalen U937-Zellen (differenzierte, humane monozytäre Zellinie mit natürlich exprimierten LTB₄-Rezeptoren) wird durch steigende Konzentration der Testsubstanz dosisabhängig inhibiert (Inkubation 2h bei 0°C). Nach Abtrennung des ungebundenen ³H-LTB₄ durch Membranfiltration wird die Radioaktivität des gebundenen LTB₄-Rezeptor/³H-LTB₄-Komplexes durch Szintilationsmessung quantifiziert.
Die Bestimmung der Affinität (Inhibitionskonstante Kᵢ) erfolgte durch iterative Anpassung einer Verdrängungskurve an die Meßwerte (Programm: "gekoppelte Massengleichgewichte" auf Wang-Computer).

### b) Aggregation von neutrophilen Granulozyten des Meerschweinchens

Indiziert durch LTB₄ in vitro (Zunahme der Lichttransmission im Aggregometer, aufgezeichnet in mm; je Experiment Doppelbestimmung): Hemmung 2 min nach Inkubation mit Prüfsubstanz in Polydiol/DMSO.

### c) Leukotrien-B₄-indizierte Neutrophilen-Akkumulation am Mäuseohr

Bewertung des neutrophilen Einstroms durch fotometrische Messung (mOD/min) der Myeloperoxidaseaktivität (Bradley et al.: J. Invest. Dermatol. 78, 206, 1982) in der Ohrhaut. Zunahme 6h nach topischer Behandlung des linken Ohres mit LTB₄ (beidseitig je 250 ng) gegenüber dem rechten Ohr (2 x 5 µl Aceton als Lösungsmittel). Substanzgabe per os in 1 %iger Tylose 300, 30 min vor LTB₄-Reiz.

### (4) Ergebnisse

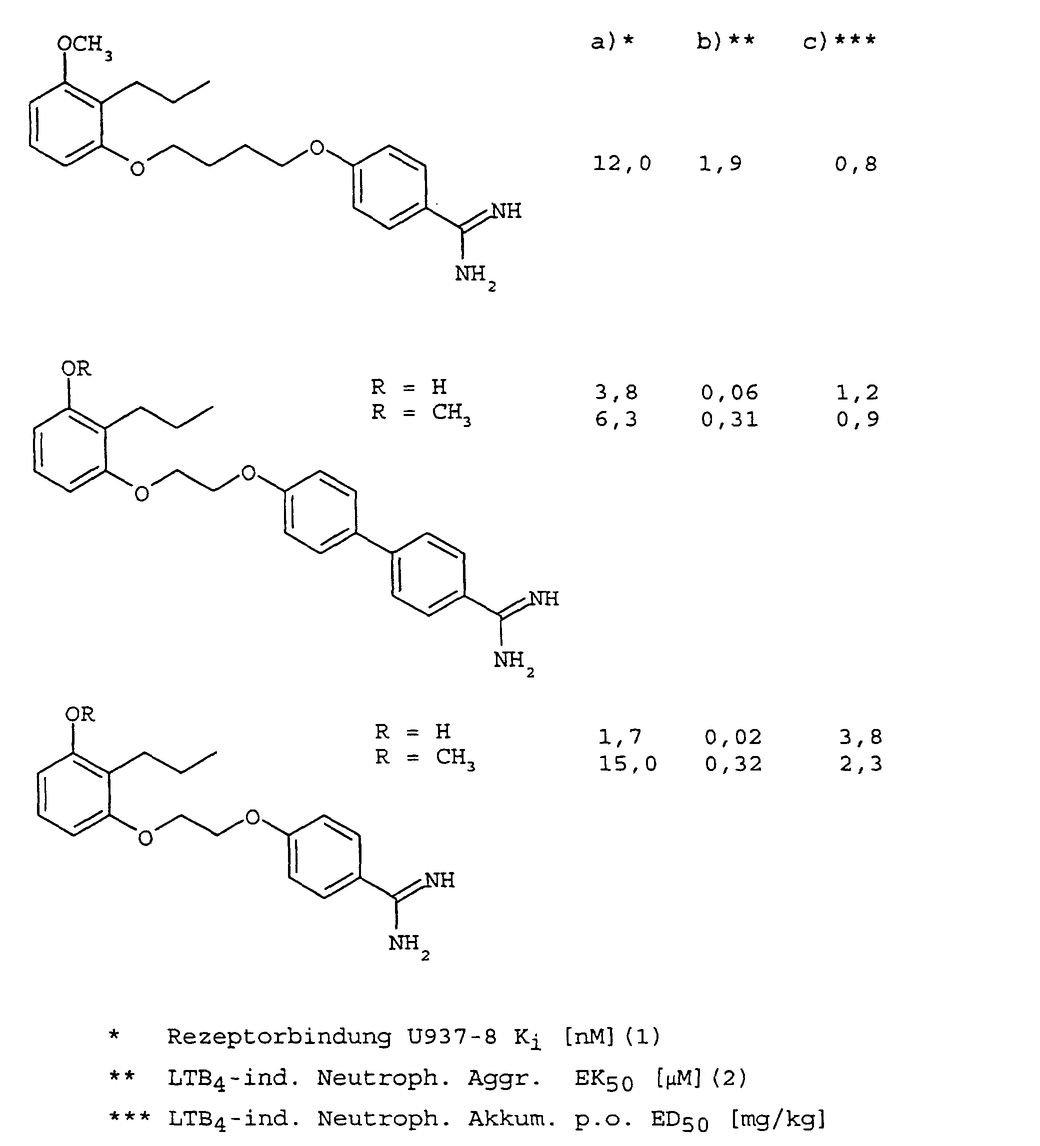

Die ³H-LTB₄-Rezeptorbindung an Meerschweinchen-Milzzellen in Gegenwart von 10 % Blutplasma lieferte Kᵢ-Werte von z.T. weit unter 1 µM, insbesondere zwischen 0,2 und 0,02.

Die Hemmung der LTB₄-induzierten Neutrophilen-Aggregation ergab EC₅₀-Werte zwischen etwa 0,5 und 0,05 µM.

Die nachstehenden Beispiele verdeutlichen die Herstellmöglichkeiten der erfindungsgemäßen Verbindungen.

### Verfahren 1:

### Referenz-Beispiel 1

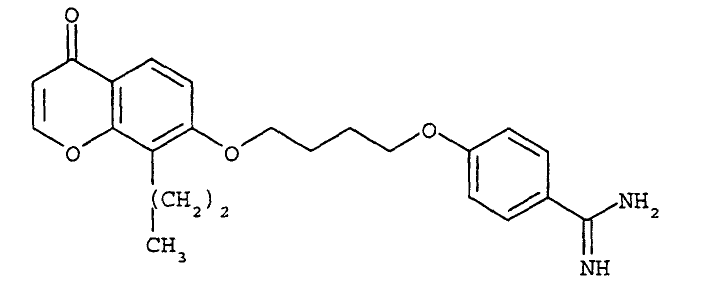

Zu einer Lösung von 2,0 g 7-[4-(4-Cyano-phenoxy)-E-but(2)-enyloxy]-8-propyl-4H-1-benzopyran-4-on in 50 ml Chloroform und 1,5 ml Ethanol gibt man 5 ml einer Lösung von Chlorwasserstoff in Diethylether (17 %). Man läßt das Gemisch 14 Tage bei Raumtemperatur stehen und fällt das Produkt mit Diethylether.
Man erhält 1,15 g 7-[4-(4-Imidacarboxyethyl-phenoxy)-E-but(2)-enyloxy]-8-propyl-4H-1-benzopyran-4-on-hydrochlorid. Der Imidoester wird mit 50 ml ethanolischer Ammoniaklösung (5 M) versetzt und 3 Stunden bei 70°C erwärmt. Man dampft das Gemisch ein und chromatographiert den Rückstand (Chloroform/Methanol 7:3, Kieselgel). Nach Umkristallisieren aus Dichlormethan/Diethylether erhält man 0,6 g 7-[4-(4-Amidino-phenoxy)-E-but(2)-enyloxy]-8-propyl-4H-1-benzopyran-4-on-hydrochlorid (Fp. 144 - 148°C).

### Beispiel

In eine Lösung von 2,5 g 4-[4-(2-propyl-3-methoxyphenoxy)- butyloxy]-benzonitril, hergestellt aus 2-Propyl-3-methoxy- phenol und 4-Brombutoxybenzonitril, in 40 ml Ethanol leitet man bei -20°C unter Rühren 1 Stunde Chlorwasserstoff ein und läßt das Gemisch 16 Stunden bei Raumtemperatur stehen.
Man destilliert das Lösungsmittel im Vakuum ab und nimmt den Rückstand in 50 ml Ethanol auf. Dazu tropft man ein Gemisch aus 14 ml ethanolischer Ammoniaklösung und 50 ml Ethanol und läßt das Gemisch 24 Stunden bei Raumtemperatur stehen. Das Lösungsmittel dampft man ab und chromatographiert den Rückstand (Chloroform/Methanol 8:2; Kieselgel 60).
Man erhält 1,8 g 4-[4-(2-propyl-3-methoxy-phenoxy)-butyloxy]-benzamidin-hydrochlorid-hemihydrat.
(Fp. 117-121°C).

### Referenz-Beispiel 2

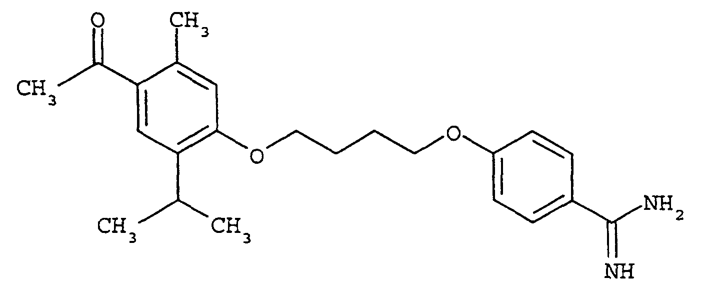

In eine Lösung von 32,0 g 4-[(4-Acetyl-2-isopropyl- 5-methyl-phenoxy)-butyloxy]-benzonitril in 350 ml Ethanol leitet man bei -20°C Chlorwasserstoff ein und rührt 48 Stunden nach. Die ausgefallenen Kristalle werden abgesaugt und mit Diethylether gewaschen. Man erhält 41,0 g 4-[4-(4-Acetyl-2-isopropyl-5-methyl-phenoxy)-butyloxy]-benzimidoethylester-hydrochlorid (Fp. 100 - 102°C Zers.). 15,0 g des Imidoesters werden bei Raumtemperatur in mehreren Portionen zu 33 ml ethanolischer Ammoniaklösung (5 M) und 100 ml Ethanol gegeben. Man läßt das Gemisch 36 Stunden bei Raumtemperatur rühren, dampft das Gemisch ein und verrührt den Rückstand mit 50 ml Wasser. Man saugt den Rückstand ab, kristallisiert aus 30 ml Ethanol um und wäscht mit Diethylether nach. Man erhält 11,5 g 4-[4-(4-Acetyl-2-isopropyl-5-methyl-phenoxy)-butyloxy]-benzamidin-hydrochlorid (Fp. 182 - 183°C Zers.).

### Referenz-Beispiel 3

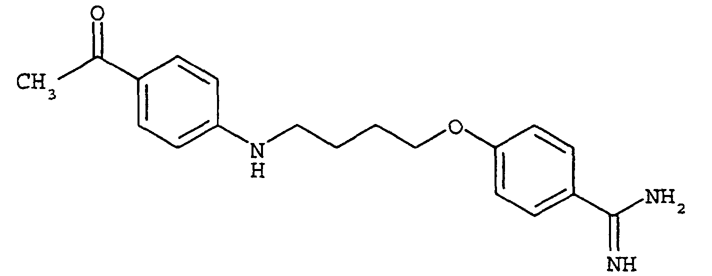

In eine Lösung von 3,0 g 4-[4-(4-Cyano-phenoxy)-butylamino]-acetophenon in 40 ml Ethanol leitet man bei -20°C unter Rühren 4 Stunden lang Chlorwasserstoff ein und läßt das Gemisch 16 Stunden bei Raumtemperatur stehen. Man destilliert das Lösungsmittel im Vakuum ab und nimmt den Rückstand in 50 ml Ethanol auf. Dazu tropft man ein Gemisch aus 14 ml ethanolischer Ammoniaklösung und 50 ml Ethanol und läßt das Gemisch 24 Stunden bei Raumtemperatur stehen. Das Lösungsmittel dampft man ab und chromatographiert den Rückstand (Chloroform/Methanol 7:3, Kieselgel 60). Man erhält 0,3 g 4-[4-(4-Amidino-phenoxy)butylamino]-acetophenon (Fp. 200 - 202°C).

### Verfahren 2:

### Referenz-Beispiel 4

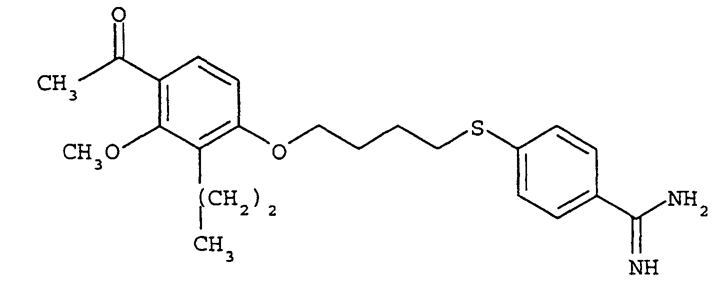

Man löst 8,2 g 4-Acetyl-3-methoxy-2-propyl-phenol in 80 ml Dimethylformamid und versetzt die Lösung portionsweise mit 1,1 g Natriumhydrid (als 80 proz. Dispersion in Weissöl). Man erwärmt das Gemisch 30 Minuten bei 80°C und versetzt mit einer Lösung von 5,75 g 4-(4-Brompropylthio)-benzamidin (hergestellt aus Dibrombutan und 4-Cyanobenzothiol über 4-(4-Brombutyl-thio)-benzonitril) in 40 ml Dimethylformamid. Nach 5 Stunden bei 80°C läßt man abkühlen, säuert mit etherischer Salzsäure an und destilliert die Lösungsmittel im Vakuum ab. Man nimmt den Rückstand in Ethanol auf und filtriert. Das Filtrat wird eingeengt. Man wiederholt den Vorgang mit Chloroform und Acetonitril. Der Rückstand wird mit Diethylether verrührt. Nach dem Abdekantieren verbleiben 5,65 g eines gelbbraunen Öls. Das Produkt wird chromatographiert (Chloroform/Methanol 7:3, Kieselgel). Man erhält 2,4 g eines Öls, das aus Toluol kristallisiert wird. Man löst das Produkt in Acetonitril, säuert mit etherischer Salzsäure an. Die Kristalle werden abgesaugt, mit kaltem Acetonitril gewaschen, in Wasser gelöst und nach Zugabe von 2 N-Salzsäure nochmals kristallisiert. Man erhält 0,8 g 4-[4-(4-Acetyl-3-methoxy-2-propylphenoxy)-butylthio]-benzamidin-hydrochlorid (Fp. 120 - 122°C).

### Verfahren 3:

### Referenz-Beispiel 5

### a) 4-[4-(4-Acetylphenoxy-butoxy]-benzamidoxim

In 300 ml Dimethylformamid werden 45,6 g (0,3 mol) 4-Hydroxybenzamidoxim und 81,3 g (0,3 mol) 4-Brom-butoxy-acetophenon gelöst. Nach Zugabe von 55,2 g (0,4 mol) wasserfreiem Kaliumcarbonat wird 2 Stunden auf 80°C erwärmt. Man saugt die anorganischen Salze ab, engt i.V. ein und kristallisiert aus Acetonitril um.
Ausbeute: 47,8 g
Fp.: 164,5 - 165,5°C.

### b) 3-[4-(4-Acetylphenoxy)butoxy]-benzamidin-Methansulfonat

47,8 g der nach a) synthetisierten Verbindung werden in der 10fachen Menge Methanol unter Zugabe der berechneten Menge Methansulfonsäure gelöst. Nach Zugabe von Raney-Nickel wird bei 5 bar bis zur beendeten Wasserstoffaufnahme hydriert. Man saugt ab, destilliert das Lösungsmittel i.V. ab und kristallisiert aus Ethanol um.
Ausbeute: 45,2 g
Fp.: 204 - 204,5°C.

Entsprechend den obigen Verfahren können die weiteren Verbindungen der Formel I erhalten werden.

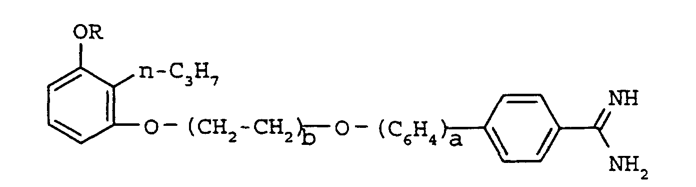

| Nr. | R | a | b | Fp. [°C] |
|---|---|---|---|---|
| 1 | H | 0 | 1 | 178-80 (Hydrochlorid) |
| 2 | H | 1 | 1 | 248-51 (Hydrochlorid) |
| 3 | H | 1 | 2 | |
| 4 | CH₃ | 0 | 1 | 176-8 (Hydrochlorid) |
| 5 | CH₃ | 1 | 1 | 236-40(Methansulfonat) |
| 6 | C₂H₅ | 0 | 1 | |
| 7 | C₂H₅ | 0 | 2 | |
| 8 | n-C₃H₇ | 0 | 2 | |
| 9 | n-C₃H₇ | 1 | 1 | |
| 10 | i-C₃H₇ | 1 | 1 | |
| 11 | n-C₄H₉ | 0 | 1 | 144-7 (Hydrochlorid) |
| 12 | n-C₄H₉ | 0 | 2 | |

## Patentansprüche

1. Verbindungen der Formel I 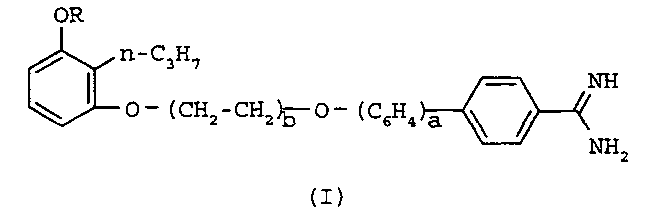 worin
a 0 oder 1,
b 1 oder 2, und
R Wasserstoff oder C₁-C₄-Alkyl bedeuten, als freie Basen oder als Säureadditionssalze, mit der Maßgabe, dass im Falle a = 0 und b = 2, R nur C₁-C₄-Alkyl bedeutet.

2. Verbindungen der Formel I, worin a = 0 oder 1/ b = 1 oder a = 0/b = 2 ist, als freie Basen oder als Säureadditionssalze.

3. Verbindungen nach Anspruch 2, wobei
R H, CH₃ oder C₂H₅, a 0 oder 1 und b 1 bedeutet, als freie Basen oder als Säureadditionssalze.

4. Verbindungen nach Anspruch 2, wobei
a 0, b 2 und R CH3 bedeuten, als freie Basen oder als Säureadditionssalze.

5. Verbindungen nach Anspruch 1 ausgewählt aus den
Verbindungen der Formel I, worin R a und b die folgenden Bedeutungen aufweisen:
| R | a | b |
|---|---|---|
| H | 0 | 1 |
| H | 1 | 1 |
| H | 1 | 2 |
| CH₃ | 0 | 1 |
| CH₃ | 1 | 1 |
| C₂H₅ | 0 | 1 |
| C₂H₅ | 0 | 2 |
| n-C₃H₇ | 0 | 2 |
| n-C₃H₇ | 1 | 1 |
| i-C₃H₇ | 1 | 1 |
| n-C₄H₉ | 0 | 1 |
| n-C₄H₉ | 0 | 2 |
als freie Basen oder als Säureadditionssalze.

6. Pharmazeutische Zubereitungen, **gekennzeichnet durch** einen Gehalt an einer Verbindung nach Anspruch 1 bis 5 in Verbindung mit üblichen Hilfs- und/oder Trägerstoffen.

7. Verwendung von Verbindungen nach Anspruch 1 bis 5 zur Herstellung von Arzneimitteln für die Behandlung von Krankheiten, bei denen LTB4-antagonistische Verbindungen eingesetzt werden können.

8. Verwendung von Verbindungen nach Anspruch 1 bis 5 bei der Herstellung von Arzneimitteln für die Behandlung von Krankheiten, bei denen entzündliche und/oder allergische Vorgänge eine Rolle spielen, insbesondere Asthma, Colitis ulcerosa, Psoriasis, und zur Behandlung einer durch nichtsteroidale Antiphlogistika induzierten Gastropathie.

9. Verfahren zur Herstellung von Verbindungen nach Anspruch 1 bis 5, **dadurch gekennzeichnet, daß** man
a) einen Imidoester der Formel 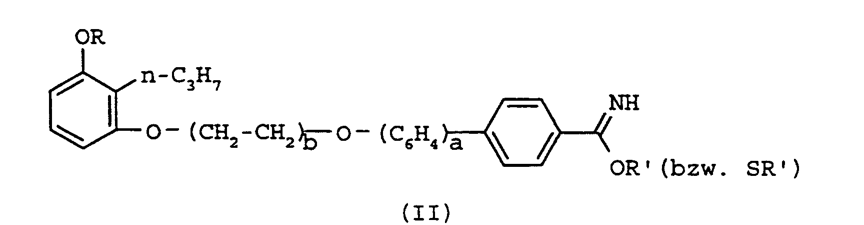 worin R' C₁-C₆-Alkyl oder Benzyl bedeutet,
und a, b und R die in Anspruch 1 bis 5 angegebene Bedeutung aufweisen, oder das entsprechende Säureimidchlorid mit Ammoniak umsetzt, oder daß man
b1) ein Phenol der Formel 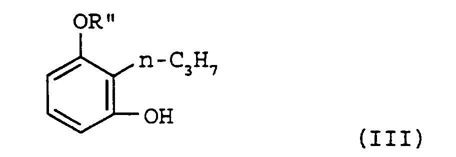 worin
R" C₁-C₄-Alkyl bedeutet, mit einer Verbindung der Formel 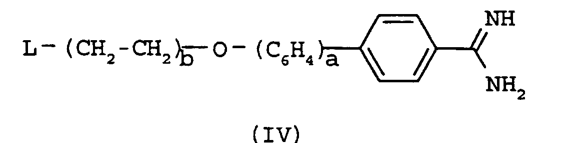 worin a und b die obige Bedeutung haben und L für eine nucleofuge Abgangsgruppe steht bzw.
b2) ein Phenol der Formel 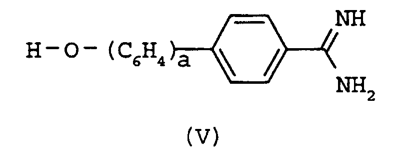 worin a die obige Bedeutung hat, mit einer Verbindung der Formel 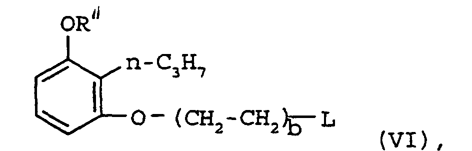 worin b, L und R" die obige Bedeutung haben, umsetzt, oder daß man
c) ein Amidoxim der Formel 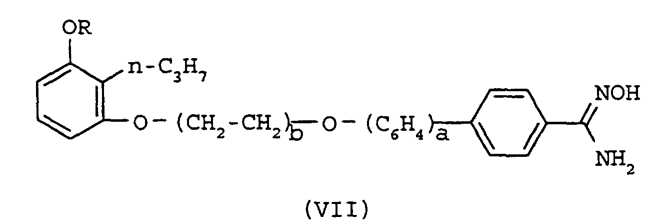 worin R, a und b die obige Bedeutung haben, zum entsprechenden Amidin reduziert
und gewünschtenfalls an erhaltenen Basen Säureadditionssalze aus erhaltenen Säureadditionssalzen die freien Basen herstellt.

## Claims

1. Compounds of formula I 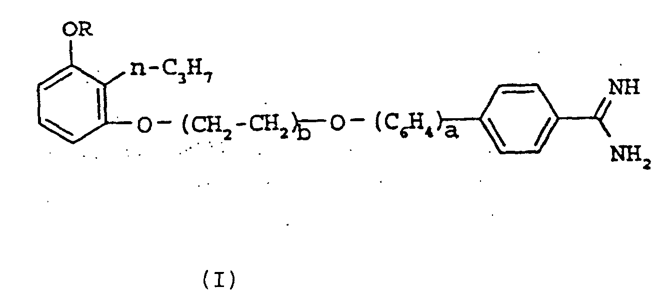 wherein
a denotes 0 or 1,
b denotes 1 or 2, and
R denotes hydrogen or C₁₋₄-alkyl, with the proviso that if a = 0 and b = 2, R can only represent C₁₋₄-alkyl.

2. Compounds of formula I, wherein a = 0 or 1/ b = 1 or a = 0/b = 2, as free bases or as acid addition salts.

3. Compounds according to claim 2, wherein R denotes H, CH₃ or C₂H₅, a denotes 0 or 1 and b denotes 1, as free bases or as acid addition salts.

4. Compounds according to claim 2, wherein a denotes 0, b denotes 2 and R denotes CH₃, as free bases or as acid addition salts.

5. Compounds according to claim 1 selected from among the compounds of formula I, wherein R, a and b have the following definitions:
| R | a | b |
|---|---|---|
| H | 0 | 1 |
| H | 1 | 1 |
| H | 1 | 2 |
| CH₃ | 0 | 1 |
| CH₃ | 1 | 1 |
| C₂H₅ | 0 | 1 |
| C₂H₅ | 0 | 2 |
| n-C₃H₇ | 0 | 2 |
| n-C₃H₇ | 1 | 1 |
| i-C₃H₇ | 1 | 1 |
| n-C₄H₉ | 0 | 1 |
| n-C₄H₉ | 0 | 2 |
as free bases or as acid addition salts.

6. Pharmaceutical compositions, **characterised in that** they contain a compound according to claims 1 to 5, in conjunction with conventional excipients and/or carriers.

7. Use of compounds according to claims 1 to 5 for preparing pharmaceutical compositions for the treatment of diseases in which LTB₄-antagonistic compounds may be used.

8. Use of compounds according to claims 1 to 5 in the preparation of pharmaceutical compositions for the treatment of diseases in which inflammatory and/or allergic processes are involved, especially asthma, ulcerative colitis and psoriasis and for treating gastropathy induced by non-steroidal antiphlogistics.

9. Process for preparing compounds according to claims 1 to 5, **characterised in that**
a) an imidoester of the formula 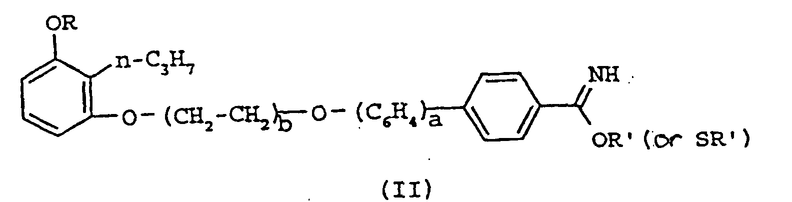 wherein R' denotes C₁₋₆-alkyl or benzyl
and a, b and R have the meanings given in claims 1 to 5, or the corresponding acid imide chloride,
is reacted with ammonia, or
b1) a phenol of the formula 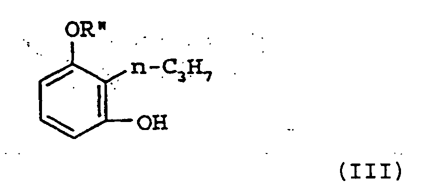 wherein R" denotes C₁₋₄-alkyl, is reacted with a compound of formula 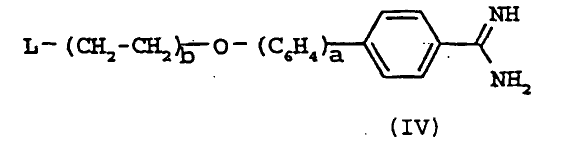 wherein a and b are as hereinbefore defined and L denotes a nucleofugic leaving group, or
b2) a phenol of formula 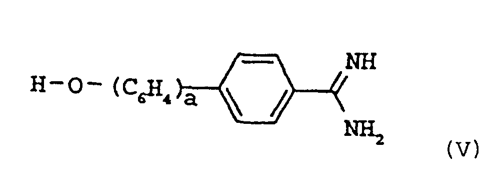 wherein a is as hereinbefore defined, is reacted with a compound of formula 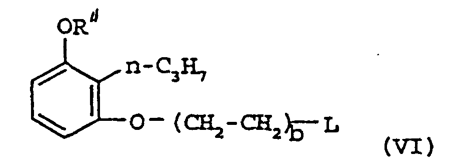 wherein b, L and R" are as hereinbefore defined;
or
c) an amidoxime of the formula 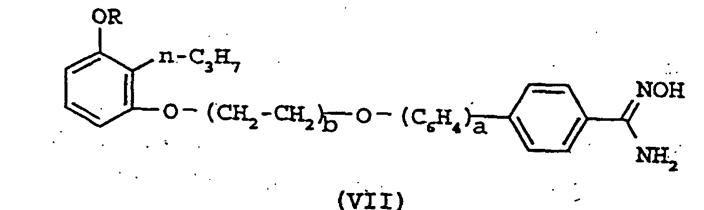
wherein R, a and b are as hereinbefore defined, is reduced to form the corresponding amidine,
and if desired acid addition salts are prepared from any bases obtained and free bases are prepared from any acid addition salts obtained.

## Revendications

1. Composés de formule I 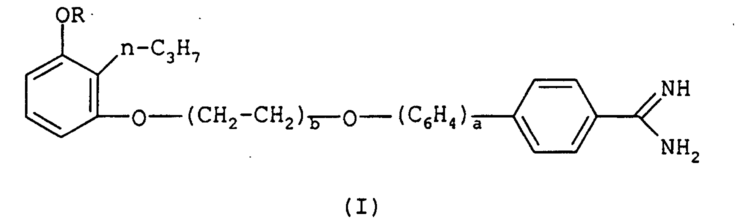 où
a représente 0 ou 1,
b représente 1 ou 2, et
R représente l'hydrogène ou alkyle en C₁-C₄, sous forme de bases libres ou de sels d'addition d'acide, à condition que, dans le cas où a = 0 et b = 2, R signifie seulement alkyle en C₁-C₄.

2. Composés de formule I où a = 0 ou 1/b = 1 ou a = 0/b = 2, sous forme de bases libres ou de sels d'addition d'acide.

3. Composés selon la revendication 2 où R représente H, CH₃ ou C₂H₅, a représente 0 ou 1 et b représente 1, sous forme de bases libres ou de sels d'addition d'acide.

4. Composés selon la revendication 2 où a représente 0, b représente 2 et R représente CH₃, sous forme de bases libres ou de sels d'addition d'acide.

5. Composés selon la revendication 1 choisis parmi les composés de formule I où R, a et b présentent les significations suivantes :
| R | a | b |
|---|---|---|
| H | 0 | 1 |
| H | 1 | 1 |
| H | 1 | 2 |
| CH₃ | 0 | 1 |
| CH₃ | 1 | 1 |
| C₂H₅ | 0 | 1 |
| C₂H₅ | 0 | 2 |
| n-C₃H₇ | 0 | 2 |
| n-C₃H₇ | 1 | 1 |
| i-C₃H₇ | 1 | 1 |
| n-C₄H₉ | 0 | 1 |
| n-C₄H₉ | 0 | 2 |
sous forme de bases libres ou de sels d'addition d'acide.

6. Préparations pharmaceutiques **caractérisées par** une teneur en un composé selon les revendications 1 à 5 en combinaison avec des adjuvants et/ou supports courants.

7. Utilisation de composés selon les revendications 1 à 5 pour la production de médicaments pour le traitement de maladies dans lesquelles des composés antagonistes de LTB₄ peuvent être utilisés.

8. Utilisation de composés selon les revendications 1 à 5 dans la production de médicaments pour le traitement de maladies dans lesquelles des processus inflammatoires et/ou allergiques jouent un rôle, en particulier l'asthme, la colite ulcéreuse, le psoriasis, et pour le traitement d'une gastropathie induite par des antiphlogistiques non stéroïdiens.

9. Procédé de production de composés selon les revendications 1 à 5 **caractérisé en ce que**
a) on fait réagir avec l'ammoniac un imidoester de formule 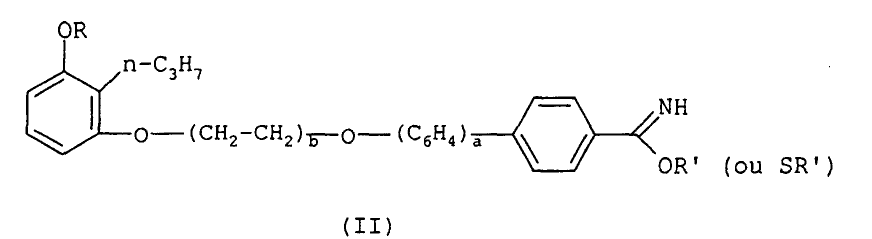 où R' représente alkyle en C₁-C₆ ou benzyle,
et a, b et R ont la signification indiquée dans les revendications 1 à 5, ou le chlorure d'imide d'acide correspondant, ou **en ce que**
b1) on fait réagir un phénol de formule 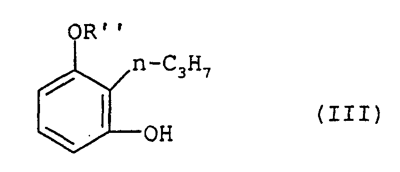 où
R" représente alkyle en C₁-C₄, avec un composé de formule 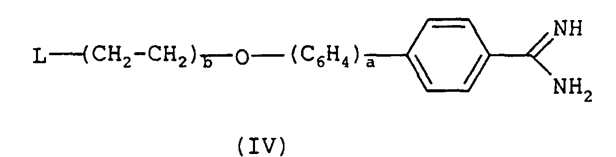 où a et b ont la signification ci-dessus et L représente un groupe partant nucléofuge ou
b2) on fait réagir un phénol de formule 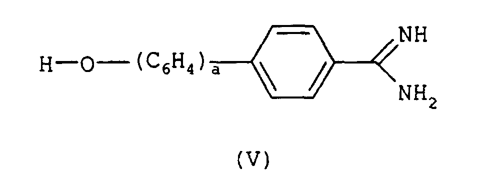 où a a la signification ci-dessus, avec un composé de formule 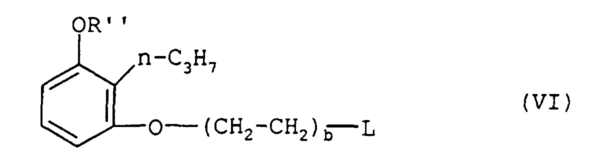 où b, L et R'' ont la signification ci-dessus, ou **en ce que**
c) on réduit une amidoxime de formule 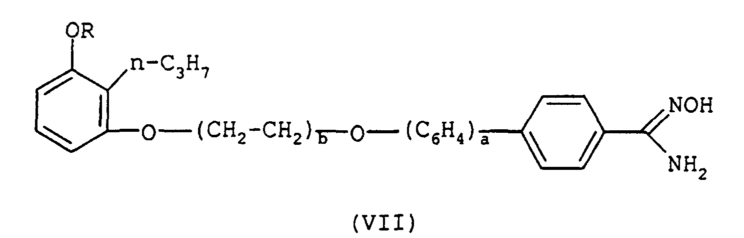
où R, a et b ont la signification ci-dessus, en l'amidine correspondante
et, si on le souhaite, on produit des sels d'addition d'acide à partir des bases obtenues et des bases libres à partir des sels d'addition d'acide obtenus.
